(19) Europäisches Patentamt
European Patent Office
Office européen des brevets

(11) **EP 4 252 625 B1**

(12) **EUROPEAN PATENT SPECIFICATION**

(45) Date of publication and mention
of the grant of the patent:
**19.02.2025 Bulletin 2025/08**

(21) Application number: **22305423.0**

(22) Date of filing: **31.03.2022**

(51) International Patent Classification (IPC):
**A61B 3/103** (2006.01)      **G02B 3/00** (2006.01)

(52) Cooperative Patent Classification (CPC):
**A61B 3/103; G02B 5/223**

(54) **DEVICE AND METHOD FOR DETERMINING A SHIFT IN A REFRACTION VALUE OF AN EYE OF A SUBJECT INDUCED BY AN OPTICAL FILTER**

VORRICHTUNG UND VERFAHREN ZUR BESTIMMUNG EINER DURCH EINEN OPTISCHEN FILTER INDUZIERTEN VERSCHIEBUNG DES BRECHUNGSWERTES EINES AUGES EINER PERSON

DISPOSITIF ET PROCÉDÉ PERMETTANT DE DÉTERMINER UN DÉCALAGE DE LA VALEUR DE RÉFRACTION D'UN IL D'UN SUJET INDUIT PAR UN FILTRE OPTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**04.10.2023 Bulletin 2023/40**

(73) Proprietor: **Essilor International
94220 Charenton-Le-Pont (FR)**

(72) Inventors:
• **DUBAIL, Marie
94410 Saint-Maurice (FR)**
• **SCHERLEN, Anne-Catherine
68470 Ranspach (FR)**
• **FRICKER, Sébastien
94000 Creteil (FR)**

(74) Representative: **Jacobacci Coralis Harle
32, rue de l'Arcade
75008 Paris (FR)**

(56) References cited:
EP-A1- 1 866 693      EP-A2- 2 018 595
JP-A- H10 339 856     US-A1- 2020 315 448

**Description**

TECHNICAL FIELD OF THE DISCLOSURE

The disclosure relates to a device and method for determining a shift in a refraction value of an eye of a subject induced by an optical filter.

BACKGROUND INFORMATION AND PRIOR ART

When a light beam is transmitted to an eye of a subject, different wavelengths of visible light are focused on different distances of the retina where the image seen by the subject is formed.

Light with long wavelengths, corresponding for example to red light, is focused on points located slightly behind the retina, while light with short wavelengths, for example blue light, is focused slightly in front of the retina. The amplitude of this defocus, i.e., the distance between the point of focus and the retina depends on the wavelength and is called "Longitudinal chromatic aberration" (LCA).

This phenomenon implies that images of different colors, i.e. of different wavelengths, or components of an image corresponding to different colors or different wavelengths may have different sharpness when seen by the eye of the subject.

Meanwhile the use of colored lenses is more and more widespread, and the colored lenses are more and more specific and complex. Colored lenses are polychromatic optical filters.

Optical filters used in eyeglasses aim to protect the ocular system and to improve comfort and visual performance in different light conditions. Colorful filters are also proposed for aesthetic purposes. They may be associated with lenses having a corrective power or not.

Because of the different focus locations of light beams having different wavelengths in the eye, the use of an optical filter that changes the wavelengths distribution of the light transmitted to the eye of the subject may modify the optimal point of focus on the retina.

Experimental results from the Applicant show that a high proportion of long wavelengths versus short wavelengths in the light reaching the eye induces a significant hyperopic shift. A high proportion of short wavelengths versus long wavelengths results in a significant myopic shift. The amplitude of the myopic shift is higher than the amplitude of the hyperopic shift.

The ametropic shift resulting from the presence of an optical filter in front of the eyes of a person can induce fatigue and reduce visual performances such as visual acuity, contrast sensitivity, reading speed.

In other words, adding an optical filter in front of the eye may induce an ametropic shift of the visual perception of the subject and may decrease the comfort and the quality of vision of the subject.

In particular, when the refraction features of the eye are determined in order to deduce the corrective power of a corrective ophthalmic lens to improve the vision of the subject, the refraction features of the eye are determined in order to focus on the retina light with wavelength comprised between green and red light, in order for the defocus associated with red light to be equivalent to the defocus associated with green light.

The refraction feature of the eye, and consequently the corrective power of the corrective ophthalmic lens, is determined with ambient natural or artificial white light, for clear lenses having negligible absorption of visible light.

The addition of an optical filter may then alter the corrective effect on the eye of the subject of the corrective ophthalmic lens thus determined.

In order to solve this problem, one could consider measuring the refractive error of the wearer with the actual spectral transmission of the chosen lenses or optical filters to allow a perfect focus of the light on the retina and produce an adapted prescription. The wearer will have a sharper vision and produce less accommodative effort.

Yet, precise methodology and precise instrument are required to get such accurate measurements and these measurements would be long and tedious.

Document US2020/315448A describes a method for determining a refraction of at least an eye of a person under specific spectral conditions.

SUMMARY OF THE INVENTION

Therefore, one object of the invention is to provide a device for determining quickly and easily the shift in the refraction value of an eye introduced by an optical filter.

The above object is achieved according to the invention by providing a device for determining a shift in a refraction value of an eye of a subject lighted by a light beam emitted by a light source, said shift being induced by an optical filter through which said light beam is transmitted to the eye of the subject, said device comprising one or more memories and one or more processors, wherein:

- said one or more memories have in memory:

  one or more values of one or more initial spectral features of said light source,
  one or more values of one or more optical features of said optical filter, and
  a refraction shift model linking a magnitude associated with said shift and one or more spectral features of the light beam transmitted to the eye of the subject; and wherein

- said one or more processors are programmed to perform the following steps:

  determining one or more values of one or more spectral features of said light beam transmitted to the eye of the subject through said optical filter based on said one or more values of one or more initial spectral features of said light source and said one or more values of one or more optical features of said optical filter; and
  determining said shift in the refraction of the eye of the subject based on said refraction shift model and said values of the spectral features of said light beam transmitted to the eye of the subject through the optical filter.

Thanks to the device according to the invention, the shift in refraction of the eye of the subject induced by an optical filter may be determined with minimal measurements, based on optical features of the optical filter that may be predetermined, known by any kind of means or measured.

Thanks to the determination of the ametropic shift with the device according to the invention, it is then possible to consider either:

- adapting the prescription based on the optical features of the optical filter to improve the quality of vision or visual comfort of the subject, for example by compensating the refraction shift induced by the filter with the power of the corrective lenses,
- using the refraction shift induced by the optical filter to improve the visual performance of the subject in specific conditions. For example, it is possible to consider using a myopic shift in near vision tasks to play the role and partly replace a spherical power of a corrective lens.

Spherical power of the ophthalmic corrective lens may be replaced by a color of this lens for specific activities of the subject.

The device according to the invention provides a tool to predict the defocus associated with an optical filter having any spectral transmission, without making any actual measurement on the subject.

Taking into account said defocus allows taking into account the corresponding ametropic shift, therefore providing a better visual performance and reducing fatigue thanks to an adapted prescription of the corrective ophthalmic lens taking into account the optical filter.

Other advantageous further features of the device according to the invention are the following:

- said one or more values of one or more initial spectral features of said light source comprise an initial spectrum of said light emitted by said light source and said one or more values of one or more optical features of said optical filter comprise values quantifying the transmission of light through said optical filter;
- said one or more processors are programmed to determine a spectrum of said light beam transmitted to the eye of the subject through said optical filter for determining said one or more values of one or more spectral features of said light beam transmitted to the eye of the subject though said optical filter;
- said one or more memories further have in memory data relative to one or more optical features of the eye of the subject and said one or more processors are programmed to take into account these data for determining one or more values of one or more spectral features of said light beam transmitted to the eye of the subject through said optical filter;
- said data relative to one or more optical features of the eye comprise data relative to the transmission of light through a front part of the eye to the retina of the eye;
- said one or more memories have in memory weighting factors representative of a light sensitivity of the retina of the eye of the subject at one or more wavelengths and wherein said one or more processors are programmed to determine said one or more values of one or more spectral features of said light beam transmitted to the eye of the subject through said optical filter taking into account these weighting factors;
- said one or more spectral features of the light beam transmitted to the eye of the subject through said optical filter comprise a statistical value of wavelength representative of the wavelength distribution of a spectrum of said light beam transmitted to the eye of the subject through said optical filter;
- said statistical value of wavelength representative of the wavelength distribution of said spectrum comprises a wavelength barycenter of said spectrum;

- said one or more memories have in memory data relative to a longitudinal chromatic aberration of the eye depending on a wavelength of a light beam transmitted to the eye and wherein said refraction shift model comprises a relationship between the shift in refraction of the eye of the subject and the longitudinal chromatic aberration of the eye of the subject at said statistical value of wavelength;
- said one or more values of one or more initial spectral features of said light source comprise an initial spectrum of said light emitted by said light source; said one or more processors are programmed to determine a reference statistical value of wavelength representative of the wavelength distribution of a reference spectrum of a reference light beam emitted by the light source and transmitted to the eye of the subject in the absence of the optical filter based on said initial spectrum of said light emitted by said light source; and said refraction shift model takes into account the longitudinal chromatic aberration of the eye of the subject at said reference statistical value of wavelength;
- said refraction shift model provides a shift value equal to the difference between longitudinal chromatic aberration of the eye at the statistical value of wavelength and the longitudinal chromatic aberration of the eye at said reference statistical value of wavelength;
- said one or more spectral features of the light beam transmitted to the eye of the subject through the optical filter comprise a spectrum of said light beam transmitted to the eye through said optical filter on a predetermined global range of wavelengths;
- said one or more memories have in memory elementary shift values associated with predetermined elementary range of wavelengths, and said refraction shift model comprises a relationship between the refraction shift and a sum, for all of said elementary range of wavelengths comprised in said predetermined global range of wavelengths, of said elementary shift values weighted by an integral of the spectrum of said light transmitted to the eye through said filter within the corresponding elementary range of wavelengths;
- said refraction shift model provides a shift value equal to a ratio between said sum and an integral of said spectrum of said light transmitted to the eye through said filter within the predetermined global range of wavelengths;
- said elementary shift values are determined taking into account a database comprising a plurality of test spectra of light transmitted to an eye and the corresponding measured values of refraction shift in said eye, by minimizing the difference between each measured value of refraction shift and a calculated value of refraction shift, said calculated value of the refraction shift being determined using said relationship and the test spectrum of light transmitted to the eye to which said measured value of refraction shift corresponds.

The invention also relates to an adaptive eyewear for a subject comprising:

- ophthalmic lenses having variable power and/or a variable filter whose variations are controlled by a controller,
- a device as described above,

wherein said device is in communication with said controller and said controller is programmed to determine a power variation and/or a tint variation of said ophthalmic lenses taking into consideration the shift in the refraction of the eye of the subject determined by said device.

The invention also relates to a method for determining a shift in a refraction value of an eye of a subject lighted by a light beam emitted by a light source, said shift being induced by an optical filter through which said light beam is transmitted to the eye of the subject, said device comprising one or more memories and one or more processors, comprising the following steps:

- determining one or more values of one or more initial spectral features of said light source,
- determining one or more values of one or more optical features of said optical filter, and
- providing a refraction shift model linking a magnitude associated with said shift and one or more spectral features of the light beam transmitted to the eye of the subject;
- determining one or more values of one or more spectral features of said light beam transmitted to the eye of the subject through said optical filter based on said one or more values of one or more initial spectral features of said light source and said one or more values of one or more optical features of said optical filter; and
- determining said shift in the refraction of the eye of the subject based on said refraction shift model and said values of the spectral features of said light beam transmitted to the eye of the subject through the optical filter.

DETAILED DESCRIPTION OF EXAMPLE(S)

The following description with reference to the accompanying drawings will make it clear what the invention consists of and how it can be achieved. The invention is not limited to the embodiments illustrated in the drawings. Accordingly, it should be understood that where features mentioned in the claims are followed by reference signs, such signs are included solely for the purpose of enhancing the intelligibility of the claims and are in no way limiting on the scope of the claims.

In the accompanying drawings:

- Figure 1 is a schematic view of a section of an eye showing examples of longitudinal chromatic aberration,
- Figure 2 is a block diagram showing the main parts of the device according to the invention and the steps of the method performed by this device,
- Figure 3 is an example graph of the radiance of a commercial light source plotted against the wavelength,
- Figure 4 is a graphical representation of the total transmittance of the clear ocular media of the human eye at different ages plotted against the wavelength,
- Figure 5 is a graphical representation of the longitudinal chromatic aberration of an eye as a function of wavelength,
- Figure 6 is a graphical representation of the response curves of the three different types of cones of the human eye as a function of wavelength, the cone response being the relative spectral sensitivity of L, M and S cones and being correlated to the probability of absorption of photons reaching the retina after filtration by the crystalline lens and macular pigment or the front part of the eye, also called anterior ocular media (no specific unit),
- Figure 7 is a graphical representation of a weighing factor of cones response as a function of wavelength,
- Figure 8 is a graphical representation of different filters transmission spectra as a function of wavelength,
- Figure 9 is a graphical representation of the ametropic shift measured and calculated by a first embodiment of the device of the invention for different filters,
- Figure 10 is a graphical representation of the ametropic shift calculated by the first embodiment device of the invention for different filters and different light sources,
- Figure 11 is a graphical representation of the wavelength ranges considered for an example of a light source spectrum in a second embodiment of the device of the invention,
- Figure 12 is a graphical representation of the ametropic shift measured and calculated by the second embodiment of the device of the invention for different filters.

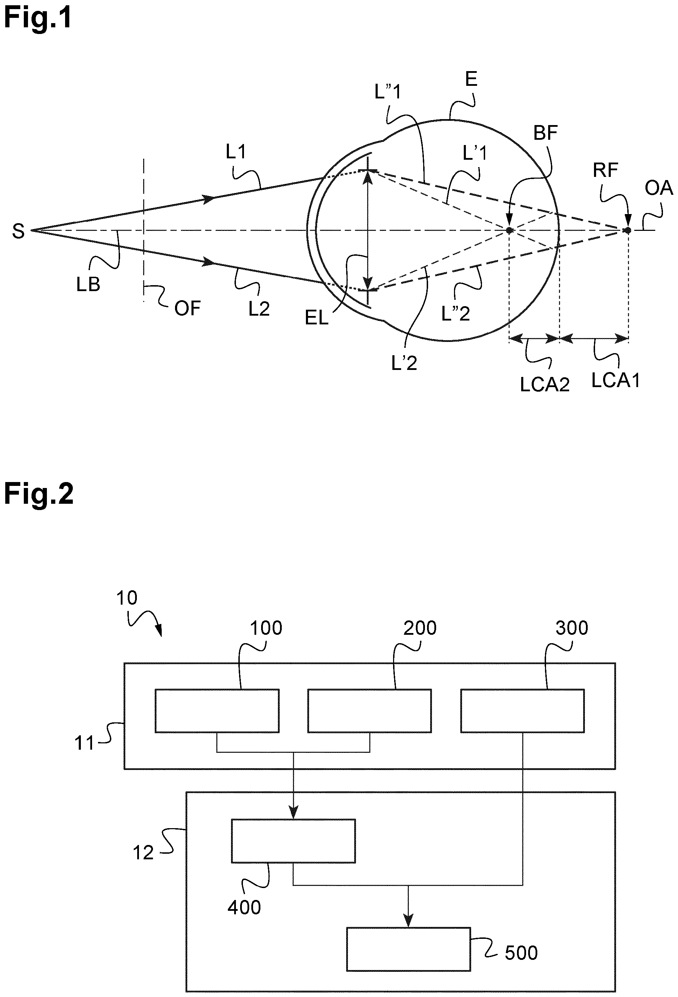

As mentioned in the introductive part and illustrated on figure 1, because of the Longitudinal Chromatic Aberration of the human eye E, light beams having different wavelengths exhibit different focus locations BF, RF of in the eye E.

Figure 1 shows the path of a light beam LB delimited by two rays of light L1, L2 incident on the eye E. The eye lens EL focuses the part of said light beam LB having shorter wavelengths, corresponding to blue colors, in front of the retina at point BF corresponding to rays of light L'1, L'2, whereas it focuses the part of said light beam LB having longer wavelengths, corresponding to red colors, behind the retina of the eye at point RF corresponding to rays of light L"1, L"2. The defocus of the red light beam is the distance LCA1 between the retina and point RF. The defocus of the blue light beam is the distance LCA2 between the retina and point BF.

The situation considered in the following is schematically shown on figure 1: the eye E of the subject receives said light beam LB emitted by a light source S having given spectral features. An optical filter OF, shown in dashed line on figure 1, is placed on the path of the light beam LB, between the light source S and the eye E.

Such an optical filter OF can comprise a tinted optical element such as a tinted substrate, or can comprise a tinted optical film associated with a base substrate that is clear or also tinted. The tint of the film/substrate can be obtained from a dye composition comprising, at least, one absorptive dye or a combination of different absorptive dyes, in order to define a colored film/substrate, a sunglass film/substrate, a filtering film/substrate filtering specific wavelength ranges of the light source such as anti-UV or blue-cut film/substrate, or a polar film/substrate.

Generally speaking, the tinted film/substrate is defined so as to reduce the visible light transmission Tv of illuminant D65 to respect specific categories of sunglasses:

- an optical element having 80% or more visible light transmission Tv, also referred to as a "category 0" optical element,
- an optical element having between 46% and 79% visible light transmission Tv, also referred to as a "category 1" optical element,
- an optical element having between 18% and 45% visible light transmission Tv, also referred to as a "category 2" optical element.

The above-described optical filter may also comprise at least one absorptive dye that is activable. The various types of activable dyes are well known to the person of ordinary skill in the art. As a result, the optical filter may be switchable between different configurations i.e. switchable between an active and an inactive states upon an external source such as an external light source. Activable dyes comprise for example photochromic or electrochromic type dyes.

The use of the optical filter OF changes the wavelengths distribution of the light beam LB transmitted to the eye E of the subject and may then globally modify the sharpness of the image seen by the subject.

The invention provides a device 10 and a methodology to predict the defocus induced by the optical filter OF for the eye E of the subject, without having to perform any measurement involving said subject.

More precisely, said device is a device 10 for determining a shift in a refraction value of the eye E of the subject lighted by the light beam LB emitted by the light source S, said shift being induced by the optical filter OF through which said light beam LB is transmitted to the eye E of the subject. This device 10 is schematically represented on figure 2. Said device 10 comprises one or more memories 11 and one or more processors 12, wherein:

-   said one or more memories 11 have in memory:

    one or more values 100 of one or more initial spectral features of said light source S,
    one or more values 200 of one or more optical features of said optical filter OF, and
    a refraction shift model 300 linking a magnitude associated with said shift and one or more spectral features of the light beam LB transmitted to the eye E of the subject; and wherein

-   said one or more processors 12 are programmed to perform the following steps:

    a) determining 400 one or more values of one or more spectral features of said light beam LB transmitted to the eye E of the subject through said optical filter OF based on said one or more values 100 of one or more initial spectral features of said light source S and said one or more values 200 of one or more optical features of said optical filter OF; and
    b) determining 500 said shift in the refraction of the eye E of the subject based on said refraction shift model 300 and said values of the spectral features of said light beam LB transmitted to the eye E of the subject through the optical filter OF.

Said one or more values 100 of one or more initial spectral features of said light source S comprise for example an initial spectrum of said light emitted by said light source S.

Said one or more values 200 of one or more optical features of said optical filter OF comprise for example values quantifying the transmission of light through said optical filter OF.

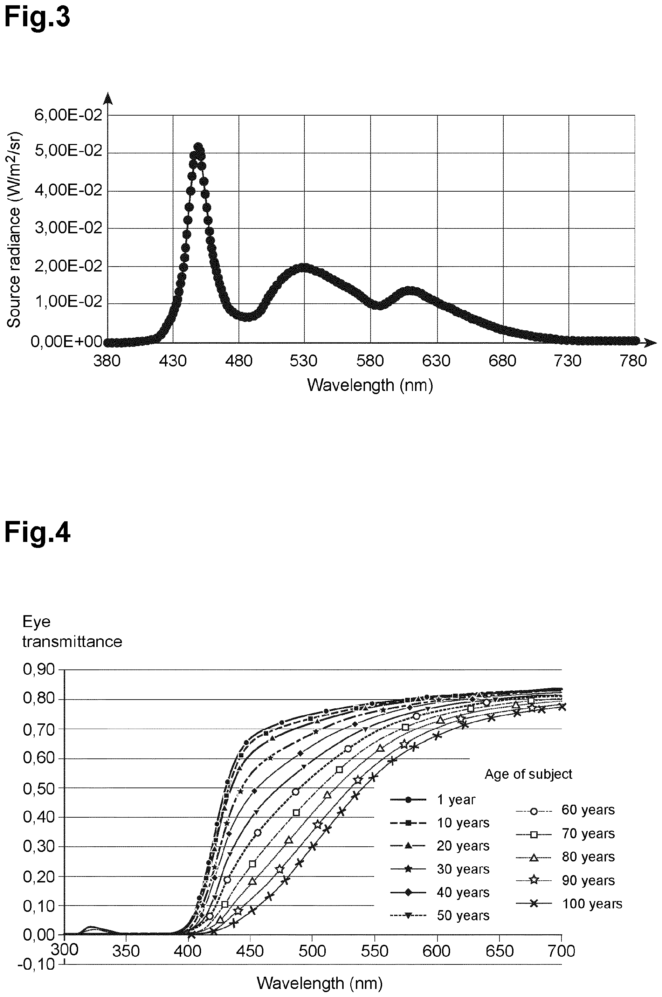

An example of the initial spectrum of said light source is shown on figure 3. This figure shows a graph of the radiance of a commercial screen as a function of wavelength. Alternatively, the initial spectrum of the light source may comprise a graph of the intensity, luminosity, luminance or any linked magnitude as a function of wavelength. The initial spectrum of the light source may also be memorized as a standard illuminant, a value of luminance or luminosity, a table of values of radiance, intensity, or other linked magnitude as a function of wavelength.

This initial spectrum may be measured thanks to a spectrometer or may be retrieved from the built-in information of said source.

Said one or more optical features of said optical filter OF may comprise a graph or a table of values of the transmission or transmittance of light through said optical filter OF.

The optical features of said optical filter OF may also comprise an opto-geometrical description of the film and/or substrate constitutive of the optical filter OF, including the geometry of the diopters, their relative positionings, and the refractive index of the material(s).

The optical features of said optical filter OF may also comprise information about the longitudinal chromatic aberration generated by the filter itself.

The value of transmittance of the filter at a given wavelength is equal to the luminance of the light beam transmitted by the filter divided by the luminance of the incident light beam at said wavelength. The value of transmittance of the filter at a given wavelength may also be defined as the radiance of the light beam transmitted by the filter divided by the radiance of the incident light beam at said wavelength.

The value of transmission of the filter at a given wavelength is equal to the intensity of the light beam transmitted by the filter divided by the intensity of the incident light beam at said wavelength.

The information about the longitudinal chromatic aberration can be provided as the material dispersion, or the value of the longitudinal chromatic aberration, or the coefficient of longitudinal chromatic aberration.

Said one or more values of one or more spectral features of said light beam LB transmitted to the eye E of the subject through said optical filter OF may comprise a spectrum of said light beam transmitted through the filter determined by multiplying the initial spectrum of the light source by the values quantifying the transmission of light through said optical filter OF at each wavelength.

The spectral features of the light beam transmitted to the eye may also comprise the amount of longitudinal chromatic aberration within the beam of light, for example within the range of the wavelengths that are included in the light beam.

In this case, said one or more processors 12 are programmed to determine in step a) a spectrum of said light beam LB transmitted to the eye E of the subject through said optical filter OF.

For example, the initial spectrum of the light source comprises intensity, luminance or radiance values for a set of wavelengths values and the values of one or more optical features of said optical filter OF comprise transmission or transmittance values of the filter for said set of wavelengths. The values of the spectral features of the light beam LB transmitted to the eye comprise the values of the intensity of the light source multiplied by the transmission of the filter for each wavelength of said set or the values of the luminance or the radiance of the light source multiplied by the transmittance of the filter for each wavelength of said set.

Naturally, other factors or parameters may be taken into account.

Optionally, said one or more memories 11 may further have in memory data relative to one or more optical features of the eye E of the subject and said one or more processors 12 are programmed to take into account these data for determining said one or more values of one or more spectral features of said light beam LB transmitted to the eye E of the subject through said optical filter OF.

Said data relative to one or more optical features of the eye E may for example comprise data relative to the transmission of light through a front part of the eye E to the retina of the eye E. The front part of the eye E corresponds to the part of the eye E located in front of the retina. This front part of the eye E indeed presents predetermined transmission features and acts as an additional optical filter placed in front of the retina, where the cells detecting the light beam are located.

In this case, said one or more processors 12 are programmed to determine in step a) a spectrum of said light beam LB transmitted to the eye E of the subject through said optical filter OF for example by multiplying the values of the intensity or luminance of the light source by the transmission or the transmittance of the filter for each wavelength of said set and by the transmission or transmittance of the front part of the eye for each wavelength.

The transmission or transmittance of the optical filter OF and/or of the front part of the eye E may be measured in a preliminary calibration step or may be retrieved from a database.

The transmission or transmittance of the front part of the eye E may comprise specific values customized for the subject, either by measures on the subject or by taking into account personal features of the subject such as the age of the subject or spectral transmission of the front part of the eye or anterior ocular media.

Examples of data relative to the total transmittance of the front part of the eye depending on wavelength for subject of different ages are presented on figure 4. The data is shown here as a graph of transmittance of the front part of the eye versus wavelength.

Alternatively, the transmission or transmittance of the front part of the eye E may comprise average transmission or transmittance values determined or a reference population of subjects or for any subject.

In a more refined embodiment, said one or more memories 11 have in memory weighting factors representative of a light sensitivity of the retina of the eye E of the subject at one or more wavelengths. Said one or more processors 12 are then programmed to determine said one or more values of one or more spectral features of said light beam LB transmitted to the eye E of the subject through said optical filter OF taking into account these weighting factors.

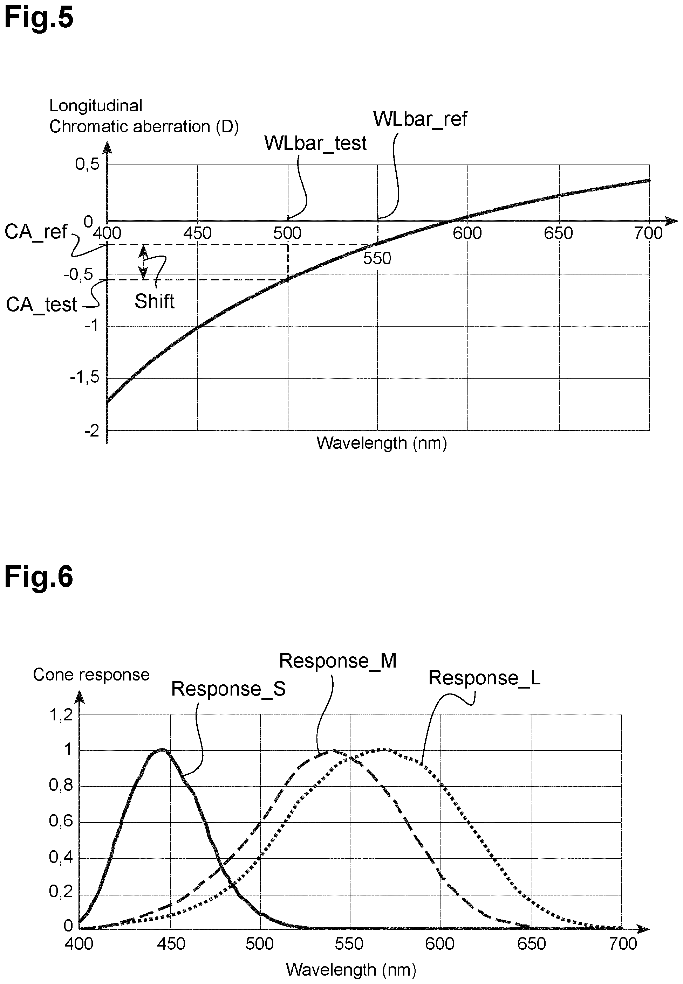

Such a weighting factor may be derived based on response curves Response_S, Response_M, Response_L of the retinal cells detecting the light beam, for example on the basis of three types of cones noted in the following S, M and L. Data relative to the response curves of the cone of the eye are available from the CIE (Commission Internationale de l'Eclairage) and is represented on figure 6 showing the light sensitivity of each type of cone depending on wavelength. Light sensitivity may be defined as the function representing the number of photons absorbed by the retina for each wavelength.

The weighting factor Wf taken into account may be determined as a weighted average of the response of the three types of cones for each wavelength. It is for example calculated as $Wf(\lambda) = ws \ast Response\_S(\lambda) + wl \ast Response\_L(\lambda) + wm \ast Response\_M(\lambda)$, where ws, wm, and wl are three scalars determining the weight of each cone response. An example showing how these scalars are determined will be developed later.

The refraction shift model 300 linking a magnitude associated with said shift and one or more spectral features of the light beam LB transmitted to the eye E of the subject may be determined by using machine learning algorithms trained on a reference database.

A preliminary step of the method according to the invention may therefore comprise building the reference database comprising measured refractive error values of reference subjects.

This reference database is for example built based on the results of a psychovisual study on a group of reference subjects. The refractive errors measured on these reference subjects presented with different optical filters with predetermined known spectral features are used to model the ametropic shift induced by a predetermined optical filter.

In the examples described below, the reference database comprises data gathered by a psychovisual study on 30 young adults. Measurements of ametropic shifts have been conducted. For each combination of a subject and an optical filter, monocular equivalent sphere (MES) was measured for each eye by a subjective refraction method. The same measurement was performed without the optical filter. The age of each subject is also known.

Two different embodiments are described in the following.

The approach used in the first embodiment uses the physiological parameters of the eye to calculate the defocus.

The approach used in the second embodiment is based on experimental data to determine a defocusing function.

First embodiment

According to this embodiment, said one or more values of one or more initial spectral features of said light source comprise an initial spectrum of said light emitted by said light source. The one or more processors 12 are programmed to determine one or more values of one or more reference spectral features of a reference light beam emitted by said light source and reaching the retina of the eye E of the subject in the absence of any added optical filter OF.

For example, the one or more processors 12 are programmed to determine a reference spectrum Spect_ref of the reference light beam. This reference spectrum Spect_ref is for example determined by multiplying the initial spectrum of the light source Spect_source by the transmission or transmittance of the front part of the eye Trans_eye, for each wavelength, or:

$$\text{Spect\_ref}(\lambda) = \text{Spect\_source}(\lambda) * \text{Trans\_eye}(\lambda).$$

The one or more processors are also programmed to determine one or more test spectral features of a test light beam emitted by said light source and reaching the retina of the eye of the subject through an added optical filter OF.

For example, the one or more processors 12 are programmed to determine a test spectrum of the test light beam. This test spectrum Spect_test is for example determined by multiplying the reference spectrum Spect_ref of the light source by the transmission or transmittance of the optical filter Trans_filter at each wavelength, or :Spect_test($\lambda$) = Spect_ref($\lambda$) * Trans_filter($\lambda$).

Said one or more spectral features of the light beam transmitted to the eye of the subject through said optical filter, i.e. the test light beam, may comprise a statistical value of wavelength representative of the wavelength distribution of the spectrum of said light beam transmitted to the eye of the subject through said optical filter, i.e. the test spectrum. In the following, this value will be named "test statistical value of wavelength".

Similarly, said one or more spectral features of the light beam transmitted to the eye E of the subject in the absence of said optical filter OF, i.e. the reference light beam, may comprise a statistical value of wavelength representative of the wavelength distribution of the reference spectrum of the reference light beam emitted by the light source and transmitted to the eye of the subject in the absence of the optical filter.

Said one or more processors 12 are programmed to determine said statistical value of wavelength representative of the wavelength distribution of the reference spectrum. In the following, this value will be named "reference statistical value of wavelength". The reference statistical value of wavelength is determined based on said initial spectrum of said light emitted by said light source.

For example, said statistical value of wavelength representative of the wavelength distribution of said test spectrum, respectively of said reference spectrum, comprises a wavelength barycenter WLbar_test, WLbar_ref of said test spectrum, respectively of said reference spectrum.

The wavelength barycenter WLbar_test, WLbar_ref may be determined as the weighted mean of the wavelengths of the spectrum, with weight factors equal to the normalized value of the spectrum at each wavelength (in intensity, luminance, radiance, luminosity....). The following formula may be used to determine said wavelength barycenter :

$$WLbar_{test} = \frac{\sum_i \lambda_i Spect_{test}(\lambda_i)}{\sum_i Spect_{test}(\lambda_i)}$$

$$WLbar_{ref} = \frac{\sum_i \lambda_i Spect_{ref}(\lambda_i)}{\sum_i Spect_{ref}(\lambda_i)}$$

In this first embodiment, said one or more memories 11 have moreover in memory data relative to a longitudinal chromatic aberration of the eye depending on a wavelength of a light beam transmitted to the eye.

An example of such data is shown on figure 5 where a graph of the longitudinal chromatic aberration of an eye in diopter versus wavelength is represented.

Alternatively, said data may comprise a table of values of the longitudinal chromatic aberration associated with the corresponding wavelength.

The longitudinal chromatic aberration of the eye put in memory may be an average longitudinal chromatic aberration for the human eye or a customized longitudinal chromatic aberration. The customized longitudinal chromatic aberration may be determined based on personal features of the subject such as age, gender, type of ametropia. It can be determined based on statistical values of longitudinal chromatic aberration for a population of subjects. The longitudinal chromatic aberration of the eye of the subject may also be measured.

Said refraction shift model then comprises a relationship between the shift in refraction of the eye of the subject and the longitudinal chromatic aberration of the eye of the subject at said statistical value of wavelength.

In practice, said refraction shift model takes into account the longitudinal chromatic aberration of the eye of the subject at said reference statistical value of wavelength.

More precisely, said refraction shift model provides a shift value S equal to the difference between longitudinal chromatic aberration LCA(WLbar_test) of the eye at the test statistical value of wavelength WLbar_test and the longitudinal chromatic aberration LCA(WLbar_ref) of the eye at said reference statistical value of wavelength WLbar_ref (see figure 5):

$$S = CA\_test - CA\_ref \text{ with } CA\_test = LCA(WL\_test) \text{ and } CA\_ref = LCA(WLbar\_ref).$$

Optionally, the refraction shift model can take into account the longitudinal chromatic aberration of the substrate of the optical filter, in addition to the longitudinal chromatic aberration of the eye. In that case, the longitudinal chromatic aberration values LCA(WLbar_test) or LCA(WLbar_ref) to consider are the sum of the longitudinal chromatic aberrations of the substrate and the longitudinal chromatic aberration of the eye.

As discussed before, as a variant, the one or more processors may be programmed to take into account a weighting factor Wf($\lambda$) for determining the reference spectrum. The weighting factor Wf($\lambda$) is determined based on the response curves of the three types of retinal cones: S, M and L (CIE data).

Such a weighting factor Wf($\lambda$) may be derived based on response curves Response_S($\lambda$), Response_M($\lambda$), Response_L($\lambda$) of the retinal cells detecting the light beam, for example on the basis of three types of cones noted in the following S, M and L.

The weighting factor Wf($\lambda$) may be determined as a weighted average of the response of the three types of cones for each wavelength. It is for example calculated as Wf($\lambda$) = ws*Response_S($\lambda$) + wl*Response_L($\lambda$) + wm*Response_M($\lambda$), where ws, wm, and wl are three scalars to be defined to determine the weight of each cone response.

For example, the one or more processors 12 are programmed to determine the reference spectrum of the reference light beam by multiplying the initial spectrum of the light source Spect_source by the transmission or transmittance of the front part of the eye Trans_eye and by the weighting factor Wf:

$$Spect\_ref(\lambda) = Spect\_source\ (\lambda)\ *Trans\_eye(\lambda)*\ Wf(\lambda).$$

The one or more processors 12 are then programmed to determine the test spectrum Spect_test taking into account the same weighting factor Wf, by multiplying the reference spectrum Spect_ref of the light source by the transmission or transmittance of the optical filter Trans_filter, or: Spect_test($\lambda$) = Spect_ref($\lambda$)* Trans_filter($\lambda$). The weighting factor Wf is taken into account in the values of the reference spectrum Spect_ref.

The weighting factor Wf($\lambda$) may be determined through the following process.

For each reference subject of a population of reference subjects and each filter of a group of reference filters, the ametropic shift is estimated through the method described above, without weighting factors. The shift in Equivalent Sphere of each eye of each subject is then measured for each filter.

Examples of implementation of these steps may be found in document US2020315448.

The values of the scalars ws, wm, and wl are then determined by optimization algorithms in order to minimize the difference between the ametropic shift estimated and the shift in Equivalent Sphere measured for each eye of each subject for each filter. In the present example, the optimization results are as follow:

$$ws = 0.05, wm = -0.5, wl=0.87.$$

The optimization space was not restricted here to positive factors.

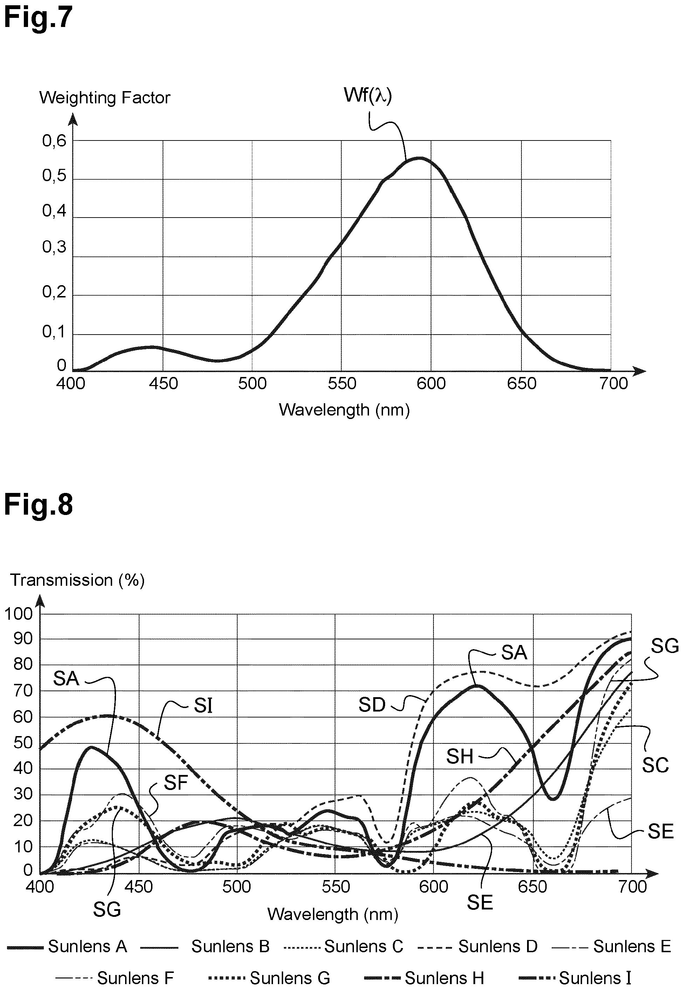

The resulting weighting factor Wf($\lambda$) is shown o figure 7. The weighting factor curve is positive everywhere.

Then, in order to test this method according to the first embodiment, the refraction shift model has been applied to a new set of 9 filters corresponding to commercial sunlenses and dopamine. A calculated shift was determined for each filter thanks to the method.

The spectra SA, SB, SC, SD, SE, SF, SG, SH, SI of each filter of the new set of 9 filters, noted sunlens or filter A to I, are shown on figure 8.

A measured value of the refraction shift introduced by each filter of this new set of filters was also measured for each reference subject of 30 reference subjects. The measured shift associated with each filter was obtained by averaging these measured values. The standard deviation of the 30 measured values was determined. The calculated shift determined with the refraction shift model was then compared with the measured shift. The results are summarized in the following table.

Table 1

| Filter | Measured shift | Calculated shift | Standard Deviation for the measured shift |
|---|---|---|---|
| Sunlens A | 0.146 | 0.077 | 0.102 |
| Sunlens B | -0.059 | 0.003 | 0.082 |
| Sunlens C | -0.043 | 0.034 | 0.095 |
| Sunlens D | 0.169 | 0.085 | 0.102 |
| Sunlens E | -0.022 | 0.027 | 0.067 |
| Sunlens F | 0.028 | 0.045 | 0.073 |
| Sunlens G | -0.029 | 0.022 | 0.077 |
| Sunlens H | 0.124 | 0.083 | 0.076 |
| Sunlens I | -0.204 | -0.218 | 0.112 |

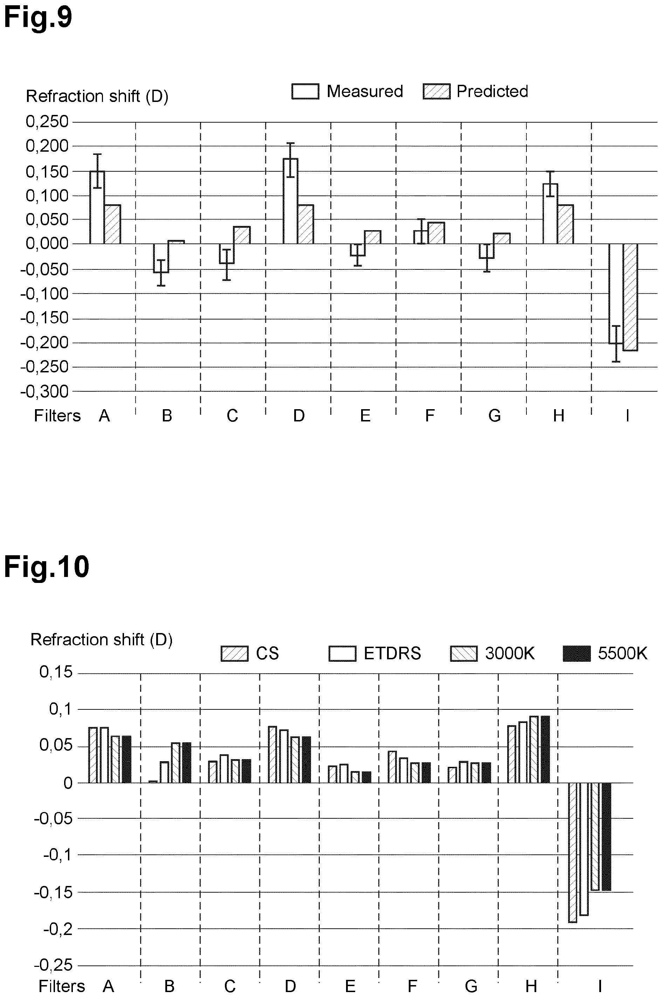

These results are also summarized on figure 9, showing the measured and calculated shift values of each filter of the new set of 9 filters. The results show that over the 9 filters, there are 5 filters that induce a smaller refraction shift and 4 filters that induce a larger refraction shift.

More precisely, sunlenses B, C, E, F, G induce a refraction shift smaller than 0.05 D.

For these filters, the average measured refraction shift is smaller than the standard deviation across all 30 reference subjects (sigma ~ 0.09 D). The average measured refraction shift is also small compared to the usual prescription tolerance in lens manufacturing, which is about 0.12 D. Finally, the average measured refraction shift is probably below the prescription sensitivity of most subjects. These refraction shifts are therefore considered to be negligible, and an accurate calculation of these shifts is not crucial.

Sunlenses A, D, H and I induce a refraction shift higher than 0.1 D.

For these filters, the average measured refraction shift is larger than the standard deviation of the measured shift values for the 30 reference subjects. Moreover, a fraction of subjects is sensitive to these values of refraction shift, meaning that they will be able to perceive it. These refraction shifts are therefore considered as significant.

For these filters, the refraction shift model described above correctly predicts the sign of the refraction shift. The average absolute error on the refraction shift is 0.05 D, which is satisfactory given the dispersion of the data.

The calculated and measured refraction shifts were here analyzed for a given light source having the spectrum shown on figure 3. This specific light source has higher radiance for determined wavelengths. To determine the influence of the light source on the calculated refraction shift, two different sunlight conditions were considered:

i) early sunrise, color temperature ~ 3000 K
ii) sun at noon, color temperature ~ 5500 K.

Other lighting conditions tested comprise a chart illuminated with a 7 LED light source referenced as ETDRS on figure 10 and the commercial screen having the spectrum of figure 3 noted CS.

The calculated refraction shift for both sunlight conditions, for the commercial screen CS having the spectrum of figure 3 and the chart illuminated with a 7 LED light source (EDTRS) were determined for a 45-year-old subject and compared.

The results are shown in figure 10. This figure 10 shows that, for the four filters A, D, H and I, the calculated refraction shift is relatively stable with respect to the light source. The calculated refraction shift may then be useful in different light conditions. The method according to the invention allows taking into account the spectral features of the light source used.

Therefore, the method described above allows determining with satisfactory accuracy the refraction shift introduced by a filter with different light sources without any measurement.

Second embodiment

According to the second embodiment, said one or more spectral features of the test light beam transmitted to the eye of the subject through the optical filter comprise a test spectrum of said test light beam on a predetermined global range of wavelengths.

This predetermined global range of wavelengths covers preferably the visible spectrum of light. It corresponds for example to the range of wavelengths comprised between 380 an 800 nm, or between 400 and 700 nm.

In this embodiment, this predetermined global range of wavelengths is divided into elementary range of wavelengths $B_i$ and an elementary shift value $S_i$ induced by each elementary range of wavelength $B_i$ is determined.

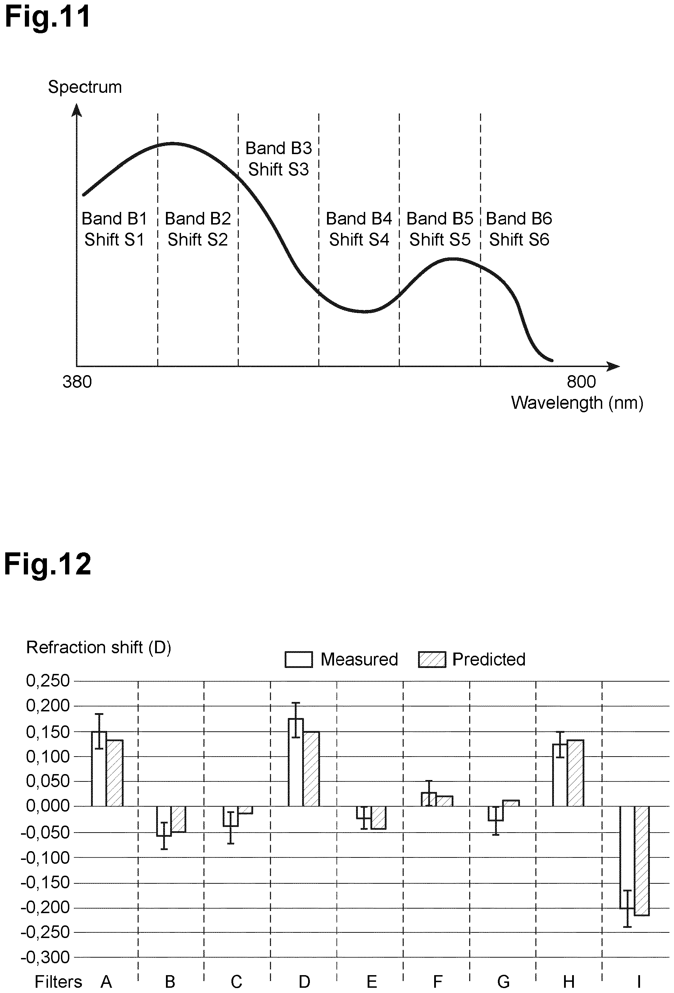

The elementary ranges of wavelengths $B_i$ correspond to bands of wavelengths, as schematically represented on figure 11.

The elementary ranges of wavelengths $B_i$ preferably cover the whole global range in a continuous manner with $B_i=[\lambda_{i1},\lambda_{i2}[=[\lambda_i,\lambda_{(i+1)}[$.

In practice, said one or more memories 11 have in memory said elementary shift values $S_i$ associated with predetermined elementary range of wavelengths $B_i$, and said refraction shift model comprises a relationship between the refraction shift and a sum, for all of said elementary range of wavelengths $B_i$ comprised in said predetermined global range of wavelengths, of said elementary shift values $S_i$ weighted by an integral of the spectrum of said light transmitted to the eye through said filter OF within the corresponding elementary range of wavelengths $B_i$.

As described in the first embodiment, for a given light source having a predetermined initial spectrum, the one or more processors 12 are programmed to determine a test spectrum of the test light beam. This test spectrum Spect_test($\lambda$) is for example determined by multiplying the reference spectrum Spect_ref($\lambda$) of the light source by the transmission or transmittance of the optical filter Trans_filter($\lambda$), or: Spect_test($\lambda$) = Spect_ref($\lambda$) * Trans_filter($\lambda$). The reference spectrum Spect_ref($\lambda$) is for example determined by multiplying the initial spectrum of the light source Spect_source($\lambda$) by the transmission or transmittance of the front part of the eye Trans_eye($\lambda$), or: Spect_ref ($\lambda$)= Spect_source($\lambda$)*Trans_eye($\lambda$), as mentioned before.

The one or more processors could also be programmed to take into account a weighting factor such as the weighting factor described in the first embodiment, when determining the reference spectrum.

The calculated refraction shift takes into account the weighted sum on the global wavelength range of the elementary shift of each band of the global wavelength range, weighted by the integral of said test spectrum in that band.

Said refraction shift model provides a shift value equal to a ratio between said sum and an integral of said spectrum of said light transmitted to the eye through said filter within the predetermined global range of wavelengths.

The one or more processors 12 are then programmed to determine the calculated refraction shift with the following formula:

$$Calculated\ refraction\ shift\ =\ \frac{\sum_i\ Si\int_{\lambda i1}^{\lambda i2}\ Spect\_test(\lambda)d\lambda}{\int_{\lambda min}^{\lambda max}\ Spect\_test(\lambda)d\lambda}$$

Said elementary shift values Si are for example determined taking into account a database comprising a plurality of test spectra of light transmitted to an eye and the corresponding measured values of refraction shift in said eye, by minimizing the difference between each measured value of refraction shift and the value of refraction shift determined using said relationship and the test spectrum of light transmitted to the eye to which said measured value of refraction shift corresponds.

In practice, as an example of implementation of this second embodiment, it is possible to define six elementary wavelength ranges of 50 nm between 400 and 700 nm (as shown on figure 11).

The elementary shift values $S_i$ are optimized to minimize the difference between calculated refraction shift and measured refraction shift using a database of measured refraction shifts. As already mentioned in the example for the first embodiment, this database may be obtained by gathering refraction shifts measured using the 9 filters described as sunlenses A to I for 30 reference subjects.

The elementary shift values $S_i$ thus obtained are summarized in the following table 2.

Table 2.

| Elementary range | Elementary shift value Si |
|---|---|
| 400 to 450 nm | - 0.287 D |
| 450 to 500 nm | - 0.078 D |
| 500 to 550 nm | - 0.659 D |
| 550 to 600 nm | 0.011 D |
| 600 to 650 nm | 0.581 D |
| 650 to 700 nm | 0.159 D |

The comparison between the measured refraction shift and the calculated refraction shift for the 9 filters "sunlens A to I" is shown on figure 12.

We note that the calculated refraction shifts are quite close to the measured ones, especially on the four filters A, D, H and I The refraction shift model used in this second embodiment of the method is accurate.

In this example, the refraction shift model has 6 degrees of freedom, namely the 6 elementary refraction shift $S_i$ values, while the refraction shift model of the first embodiment only had 3 degrees of freedom, namely the 3 scalars of the weight factor.

In another example of the second embodiment, it is possible to define three elementary wavelength ranges of 100 nm between 400 and 700 nm.

The elementary shift values $S_i'$ then determined are summarized in the following table 3.

Table 3.

| Elementary range | Elementary shift value Si' |
| --- | --- |
| 400 to 500 nm | - 0.160 D |
| 500 to 600 nm | - 0.548 D |
| 600 to 700 nm | 0.580 D |

The results are almost as satisfactory with 3 elementary wavelength ranges as with 6 elementary wavelength ranges. The difference between the measured refraction shifts and the refraction shift calculated according to the method of the invention is less than 0.04 D for all filters.

Optionally, the refraction shift model can take into account the longitudinal chromatic aberration of the substrate of the optical filter, in addition to the longitudinal chromatic aberration of the eye. In that case, the elementary shift values Si need to be established for multiple substrate materials. When predicting the refraction shift for a given optical filter, the Si values corresponding to the specific substrate of the optical material should be used.

The device and methods of the invention therefore allow determining easily, quickly and without having to perform any measurement the refraction shift introduced by a filter OF for the eye E of a subject.

The refraction shift calculated thanks to the device and method described may be used for many different applications.

It may be used to modify the power of a corrective lens including a filter to compensate this refraction shift and ensure an accurate visual correction.

The power of the corrective lens may be fixed : it is then modified before manufacturing the eyewear. In this case, the device according to the invention can be embedded into the lens manufacturing calculator that designs a lens according to the chosen optical filter features.

It can also be modified in real time in the case of ophthalmic lenses having variable power and/or variable filter included. In this case, the power variation and/or the tint variation is adapted taking in consideration the refraction shift induced by the pending tint of the optical filter. The device according to the invention can be embedded in the frame wearing the ophthalmic lenses having variable power and/or variable filter or in any device adapted to communicate with a controller controlling of said ophthalmic lenses having variable power and/or variable filter, preferably wearable by the subject.

The invention also relates to an adaptive eyewear for the subject. The eyewear comprises:

- ophthalmic lenses having variable power and/or a variable filter whose variations are controlled by a controller,
- said device according to the invention and described above,

wherein said device is in communication with said controller and said controller is programmed to determine a power variation and/or a tint variation of said ophthalmic lenses taking into consideration the shift in the refraction of the eye of the subject determined by said device.

Further, the refraction shift model could be modified to take into account the index of the lens substrate and/or of the material of the filter.

**Claims**

1. Device (10) for determining a shift in a refraction value of an eye (E) of a subject lighted by a light beam emitted by a light source (S), said shift being induced by an optical filter (OF) through which said light beam is transmitted to the eye (E) of the subject, said device (10) comprising one or more memories (11) and one or more processors (12), wherein:

- said one or more memories (11) have in memory:

one or more values (100) of one or more initial spectral features of said light source (S),
one or more values (200) of one or more optical features of said optical filter (OF), and
a refraction shift model (300) linking a magnitude associated with said shift and one or more spectral features of the light beam transmitted to the eye (E) of the subject; and wherein

- said one or more processors (12) are programmed to perform the following steps:

determining (400) one or more values of one or more spectral features of said light beam transmitted to the eye of the subject through said optical filter (OF) based on said one or more values of one or more initial spectral features of said light source (S) and said one or more values of one or more optical features of said optical filter (OF); and
determining (500) said shift in the refraction of the eye (E) of the subject based on said refraction shift model and said values of the spectral features of said light beam transmitted to the eye (E) of the subject through the optical filter (OF).

2. Device according to claim 1, wherein said one or more values of one or more initial spectral features of said light source (S) comprise an initial spectrum of said light emitted by said light source (S) and said one or more values of one or more optical features of said optical filter (OF) comprise values quantifying the transmission of light through said optical filter (OF).

3. Device according to any one of claim 1 and 2, wherein said one or more processors (12) are programmed to determine a spectrum of said light beam transmitted to the eye (E) of the subject through said optical filter (OF) for determining said one or more values of one or more spectral features of said light beam transmitted to the eye of the subject though said optical filter.

4. Device according to any one of claims 1 to 3, wherein said one or more memories (11) further have in memory data relative to one or more optical features of the eye (E) of the subject and said one or more processors are programmed to take into account these data for determining one or more values of one or more spectral features of said light beam transmitted to the eye (E) of the subject through said optical filter (OF).

5. Device according to claim 4, wherein said one or more memories(11) have in memory weighting factors (Wf) representative of a light sensitivity of the retina of the eye (E) of the subject at one or more wavelengths and wherein said one or more processors (12) are programmed to determine said one or more values of one or more spectral features of said light beam transmitted to the eye (E) of the subject through said optical filter (OF) taking into account these weighting factors (Wf).

6. Device according to any one of claims 1 to 5, wherein said one or more spectral features of the light beam transmitted to the eye (E) of the subject through said optical filter (OF) comprise a statistical value of wavelength representative of the wavelength distribution of a spectrum of said light beam transmitted to the eye (E) of the subject through said optical filter (OF).

7. Device according to claim 6, wherein said statistical value of wavelength representative of the wavelength distribution of said spectrum comprises a wavelength barycenter of said spectrum.

8. Device according to any one of claims 6 and 7, wherein said one or more memories (11) have in memory data relative to a longitudinal chromatic aberration of the eye depending on a wavelength of a light beam transmitted to the eye (E) and wherein said refraction shift model comprises a relationship between the shift in refraction of the eye of the subject and the longitudinal chromatic aberration of the eye of the subject at said statistical value of wavelength.

9. Device according to claim 8, wherein:

- said one or more values of one or more initial spectral features of said light source comprise an initial spectrum of said light emitted by said light source (S);
- said one or more processors (12) are programmed to determine a reference statistical value of wavelength representative of the wavelength distribution of a reference spectrum of a reference light beam emitted by the light source (S) and transmitted to the eye of the subject in the absence of the optical filter (OF) based on said initial spectrum of said light emitted by said light source (S); and
- said refraction shift model takes into account the longitudinal chromatic aberration of the eye (E) of the subject at said reference statistical value of wavelength.

10. Device according to claim 9, wherein said refraction shift model provides a shift value equal to the difference between longitudinal chromatic aberration of the eye at the statistical value of wavelength and the longitudinal chromatic aberration of the eye at said reference statistical value of wavelength.

11. Device according to any one of claims 1 to 5, wherein said one or more spectral features of the light beam transmitted to the eye (E) of the subject through the optical filter (OF) comprise a spectrum of said light beam transmitted to the eye (E) through said optical filter (OF) on a predetermined global range of wavelengths.

12. Device according to claim 11, wherein said one or more memories (11) have in memory elementary shift values ($S_i$) associated with predetermined elementary range of wavelengths ($B_i$), and said refraction shift model comprises a relationship between the refraction shift and a sum, for all of said elementary range of wavelengths ($B_i$) comprised in said predetermined global range of wavelengths, of said elementary shift values ($S_i$) weighted by an integral of the spectrum of said light transmitted to the eye through said filter within the corresponding elementary range of wavelengths ($B_i$).

13. Device according to claim 12, wherein said refraction shift model provide a shift value equal to a ratio between said sum and an integral of said spectrum of said light transmitted to the eye (E) through said optical filter (OF) within the predetermined global range of wavelengths.

14. Device according to any one of claims 12 and 13, wherein said elementary shift values ($S_i$) are determined taking into account a database comprising a plurality of test spectra of light transmitted to an eye and the corresponding measured values of refraction shift in said eye, by minimizing the difference between each measured value of refraction shift and a calculated value of refraction shift, said calculated value of the refraction shift being determined using said relationship and the test spectrum of light transmitted to the eye to which said measured value of refraction shift corresponds.

15. Adaptive eyewear for a subject comprising :

- ophthalmic lenses having variable power and/or a variable filter whose variations are controlled by a controller,
- a device according to any one of claims 1 to 14,

wherein said device is in communication with said controller and said controller is programmed to determine a power variation and/or a tint variation of said ophthalmic lenses taking into consideration the shift in the refraction of the eye (E) of the subject determined by said device.

16. Method for determining a shift in a refraction value of an eye (E) of a subject lighted by a light beam emitted by a light source (S), said shift being induced by an optical filter (OF) through which said light beam is transmitted to the eye (E) of the subject, said device comprising one or more memories (11) and one or more processors (12), comprising the following steps:

- determining one or more values (100) of one or more initial spectral features of said light source,
- determining one or more values (200) of one or more optical features of said optical filter, and
- providing a refraction shift model (300) linking a magnitude associated with said shift and one or more spectral features of the light beam transmitted to the eye of the subject;
- determining (400) one or more values of one or more spectral features of said light beam transmitted to the eye (E) of the subject through said optical filter (OF) based on said one or more values of one or more initial spectral features of said light source (S) and said one or more values of one or more optical features of said optical filter (OF); and
- determining (500) said shift in the refraction of the eye (E) of the subject based on said refraction shift model and said values of the spectral features of said light beam transmitted to the eye (E) of the subject through the optical filter (OF).

**Patentansprüche**

1. Vorrichtung (10) zum Bestimmen einer Verschiebung eines Brechungswertes eines Auges (E) eines Patienten, das durch einen Lichtstrahl beleuchtet wird, der durch eine Lichtquelle (S) emittiert wird, wobei die Verschiebung durch einen optischen Filter (OF) induziert wird, durch den der Lichtstrahl zum Auge (E) des Patienten übertragen wird, wobei die Vorrichtung (10) einen oder mehrere Speicher (11) und einen oder mehrere Prozessoren (12) umfasst, wobei:

   - der eine oder die mehreren Speicher (11) Folgendes im Speicher enthalten:

      einen oder mehrere Werte (100) von einem oder mehreren ursprünglichen spektralen Merkmalen der Lichtquelle (S), einen oder mehrere Werte (200) von einem oder mehreren optischen Merkmalen des optischen Filters (OF), und
      ein Brechungsverschiebungsmodell (300), das eine Größe, die der Verschiebung zugeordnet ist, und ein oder mehrere spektrale Merkmale des Lichtstrahls, der zum Auge (E) des Patienten übertragen wird, verbindet; und wobei

   - der eine oder die mehreren Prozessoren (12) programmiert sind, die folgenden Schritte durchzuführen:

      Bestimmen (400) eines oder mehrerer Werte von einem oder mehreren spektralen Merkmalen des Lichtstrahls, der durch den optischen Filter (OF) zum Auge des Patienten übertragen wird, auf der Grundlage des einen oder der mehreren Werte von einem oder mehreren ursprünglichen spektralen Merkmalen der Lichtquelle (S) und des einen oder der mehreren Werte von einem oder mehreren optischen Merkmalen des optischen Filters (OF); und
      Bestimmen (500) der Verschiebung der Brechung des Auges (E) des Patienten auf der Grundlage des Brechungsverschiebungsmodells und der Werte der spektralen Merkmale des Lichtstrahls, der durch den optischen Filter (OF) zum Auge (E) des Patienten übertragen wird.

2. Vorrichtung nach Anspruch 1, wobei der eine oder die mehreren Werte von einem oder mehreren ursprünglichen spektralen Merkmalen des Lichtstrahls (S) ein ursprüngliches Spektrum des Lichts, das durch die Lichtquelle (S) emittiert wird, umfassen und der eine oder die mehreren Werte von einem oder mehreren optischen Merkmalen des optischen Filters (OF) Werte umfassen, die die Übertragung von Licht durch den optischen Filter (OF) quantifizieren.

3. Vorrichtung nach einem der Ansprüche 1 und 2, wobei der eine oder die mehreren Prozessoren (12) programmiert sind, zum Bestimmen des einen oder der mehreren Werte von einem oder mehreren spektralen Merkmalen des Lichtstrahls, der durch den optischen Filter zum Auge des Patienten übertragen wird, ein Spektrum des Lichtstrahls, der durch den optischen Filter (OF) zum Auge (E) des Patienten übertragen wird, zu bestimmen.

4. Vorrichtung nach einem der Ansprüche 1 bis 3, wobei der eine oder die mehreren Speicher (11) ferner Daten in Bezug auf ein oder mehrere optische Merkmale des Auges (E) des Patienten im Speicher aufweisen und der eine oder die mehreren Prozessoren programmiert sind, diese Daten zum Bestimmen eines oder mehrerer Werte von einem oder mehreren spektralen Merkmalen des Lichtstrahls, der durch den optischen Filter (OF) zum Auge (E) des Patienten übertragen wird, zu berücksichtigen.

5. Vorrichtung nach Anspruch 4, wobei der eine oder die mehreren Speicher (11) Gewichtungsfaktoren (Wf), die eine Lichtempfindlichkeit der Retina des Auges (E) des Patienten bei einer oder mehreren Wellenlängen darstellen, im Speicher aufweisen und wobei der eine oder die mehreren Prozessoren (12) programmiert sind, den einen oder die mehreren Werte von einem oder mehreren spektralen Merkmalen des Lichtstrahls, der durch den optischen Filter (OF) zum Auge (E) des Patienten übertragen wird, unter Berücksichtigung dieser Gewichtungsfaktoren (Wf) zu bestimmen.

6. Vorrichtung nach einem der Ansprüche 1 bis 5, wobei das eine oder die mehreren spektralen Merkmale des Lichtstrahls, der durch den optischen Filter (OF) zum Auge (E) des Patienten übertragen wird, einen statistischen Wellenlängenwert, der die Wellenlängenverteilung eines Spektrums des Lichtstrahls, der durch den optischen Filter (OF) zum Auge (E) des Patienten übertragen wird, darstellt, umfassen.

7. Vorrichtung nach Anspruch 6, wobei der statistische Wellenlängenwert, der die Wellenlängenverteilung des Spektrums darstellt, einen Wellenlängenschwerpunkt des Spektrums umfasst.

8. Vorrichtung nach einem der Ansprüche 6 und 7, wobei der eine oder die mehreren Speicher (11) Daten in Bezug auf eine chromatische Längsaberration des Auges abhängig von einer Wellenlänge eines Lichtstrahls, der zum Auge (E) übertragen wird, im Speicher aufweisen und wobei das Brechungsverschiebungsmodell eine Relation zwischen der Verschiebung der Brechung des Auges des Patienten und der chromatischen Längsaberration des Auges des Patienten bei dem statistischen Wellenlängenwert umfasst.

9. Vorrichtung nach Anspruch 8, wobei:

   - der eine oder die mehreren Werte von einem oder mehreren ursprünglichen spektralen Merkmalen der Lichtquelle ein ursprüngliches Spektrum des Lichts, das durch die Lichtquelle (S) emittiert wird, umfassen;
   - der eine oder die mehreren Prozessoren (12) programmiert sind, einen statistischen Referenzwellenlängenwert, der die Wellenlängenverteilung eines Referenzspektrums eines Referenzlichtstrahls, der durch die Lichtquelle (S) emittiert wird und ohne den optischen Filter (OF) zum Auge des Patienten übertragen wird, darstellt, auf der Grundlage des ursprünglichen Spektrums des Lichts, das durch die Lichtquelle (S) emittiert wird, zu bestimmen; und
   - das Brechungsverschiebungsmodell die chromatische Längsaberration des Auges (E) des Patienten bei dem statistischen Referenzwellenlängenwert berücksichtigt.

10. Vorrichtung nach Anspruch 9, wobei das Brechungsverschiebungsmodell einen Verschiebungswert bereitstellt, der gleich der Differenz zwischen der chromatischen Längsaberration des Auges bei dem statistischen Wellenlängenwert und der chromatischen Längsaberration des Auges bei dem statistischen Referenzwellenlängenwert ist.

11. Vorrichtung nach einem der Ansprüche 1 bis 5, wobei das eine oder die mehreren spektralen Merkmale des Lichtstrahls, der durch den optischen Filter (OF) zum Auge (E) des Patienten übertragen wird, ein Spektrum des Lichtstrahls, der durch den optischen Filter (OF) zum Auge (E) des Patienten übertragen wird, auf einem vorgegebenen globalen Bereich von Wellenlängen umfassen.

12. Vorrichtung nach Anspruch 11, wobei der eine oder die mehreren Speicher (11) Elementarverschiebungswerte (Si), die einem vorgegebenen Elementarbereich von Wellenlängen (Bi) zugeordnet sind, im Speicher aufweisen und das Brechungsverschiebungsmodell eine Relation zwischen der Brechungsverschiebung und einer Summe für alle des Elementarbereichs von Wellenlängen (Bi), die in dem vorgegebenen globalen Bereich von Wellenlängen umfasst sind, der Elementarverschiebungswerte (Si), die durch ein Integral des Spektrums des Lichts, das durch den Filter zum Auge übertragen wird, in dem entsprechenden Elementarbereich von Wellenlängen (Bi) gewichtet sind, umfasst.

13. Vorrichtung nach Anspruch 12, wobei das Brechungsverschiebungsmodell einen Verschiebungswert bereitstellt, der gleich einem Verhältnis zwischen der Summe und einem Integral des Spektrums des Lichts, das durch den optischen Filter (OF) zum Auge (E) übertragen wird, in dem vorgegebenen globalen Bereich von Wellenlängen ist.

14. Vorrichtung nach einem der Ansprüche 12 und 13, wobei die Elementarverschiebungswerte (Si) unter Berücksichtigung einer Datenbank, die mehrere Prüfspektren von Licht, das zu einem Auge übertragen wird, und die entsprechenden Messwerte einer Brechungsverschiebung im Auge umfasst, durch Minimieren der Differenz zwischen jedem Messwert der Brechungsverschiebung und einem berechneten Wert der Brechungsverschiebung bestimmt werden, wobei der berechnete Wert der Brechungsverschiebung unter Verwendung der Relation und des Prüfspektrums von Licht, das zum Auge übertragen wird, dem der Messwert der Brechungsverschiebung entspricht, bestimmt wird.

15. Anpassbare Sehhilfe für einen Patienten, die Folgendes umfasst:

   - optische Linsen, die eine veränderliche Stärke und/oder einen veränderlichen Filter aufweisen, deren Veränderungen durch eine Steuereinheit gesteuert werden,
   - eine Vorrichtung nach einem der Ansprüche 1 bis 14, wobei sich die Vorrichtung mit der Steuereinheit in Kommunikation befindet und die Steuereinheit programmiert ist, eine Stärkenveränderung und/oder eine Farbtonveränderung der optischen Linsen unter Berücksichtigung der Verschiebung der Brechung des Auges (E) des Patienten, die durch die Vorrichtung bestimmt wird, zu bestimmen.

16. Verfahren zum Bestimmen einer Verschiebung eines Brechungswertes eines Auges (E) eines Patienten, das durch einen Lichtstrahl beleuchtet wird, der durch eine Lichtquelle (S) emittiert wird, wobei die Verschiebung durch einen optischen Filter (OF) induziert wird, durch den der Lichtstrahl zum Auge (E) des Patienten übertragen wird, wobei die Vorrichtung einen oder mehrere Speicher (11) und einen oder mehrere Prozessoren (12) umfasst, das die folgenden Schritte umfasst:

- Bestimmen eines oder mehrerer Werte (100) von einem oder mehreren ursprünglichen spektralen Merkmalen der Lichtquelle,
- Bestimmen eines oder mehrerer Werte (200) von einem oder mehreren optischen Merkmalen des optischen Filters, und
- Bereitstellen eines Brechungsverschiebungsmodells (300), das eine Größe, die der Verschiebung zugeordnet ist, und ein oder mehrere spektrale Merkmale des Lichtstrahls, der zum Auge des Patienten übertragen wird, verbindet;
- Bestimmen (400) eines oder mehrerer Werte von einem oder mehreren spektralen Merkmalen des Lichtstrahls, der durch den optischen Filter (OF) zum Auge (E) des Patienten übertragen wird, auf der Grundlage des einen oder der mehreren Werte von einem oder mehreren ursprünglichen spektralen Merkmalen der Lichtquelle (S) und des einen oder der mehreren Werte von einem oder mehreren optischen Merkmalen des optischen Filters (OF); und
- Bestimmen (500) der Verschiebung der Brechung des Auges (E) des Patienten auf der Grundlage des Brechungsverschiebungsmodells und der Werte der spektralen Merkmale des Lichtstrahls, der durch den optischen Filter (OF) zum Auge (E) des Patienten übertragen wird.

## Revendications

1. Dispositif (10) de détermination d'un décalage d'une valeur de réfraction d'un œil (E) d'un sujet éclairé par un faisceau lumineux émis par une source lumineuse (S), ledit décalage étant induit par un filtre optique (OF) au travers duquel ledit faisceau lumineux est transmis à l'œil (E) du sujet, ledit dispositif (10) comprenant une ou plusieurs mémoires (11) et un ou plusieurs processeurs (12), dans lequel :

   - lesdites une ou plusieurs mémoires (11) ont en mémoire :

      une ou plusieurs valeurs (100) d'une ou de plusieurs caractéristiques spectrales initiales de ladite source lumineuse (S),
      une ou plusieurs valeurs (200) d'une ou de plusieurs caractéristiques optiques dudit filtre optique (OF), et un modèle de décalage de réfraction (300) liant une grandeur associée audit décalage et une ou plusieurs caractéristiques spectrales du faisceau lumineux transmis à l'œil (E) du sujet ; et dans lequel

   - lesdits un ou plusieurs processeurs (12) sont programmés pour réaliser les étapes suivantes :

      détermination (400) d'une ou de plusieurs valeurs d'une ou de plusieurs caractéristiques spectrales dudit faisceau lumineux transmis à l'œil du sujet au travers dudit filtre optique (OF) sur la base desdites une ou plusieurs valeurs d'une ou de plusieurs caractéristiques spectrales initiales de ladite source lumineuse (S) et desdites une ou plusieurs valeurs d'une ou de plusieurs caractéristiques optiques dudit filtre optique (OF) ; et détermination (500) dudit décalage de la réfraction de l'œil (E) du sujet sur la base dudit modèle de décalage de réfraction et desdites valeurs des caractéristiques spectrales dudit faisceau lumineux transmis à l'œil (E) du sujet au travers du filtre optique (OF).

2. Dispositif selon la revendication 1, dans lequel lesdites une ou plusieurs valeurs d'une ou de plusieurs caractéristiques spectrales initiales de ladite source lumineuse (S) comprennent un spectre initial de ladite lumière émise par ladite source lumineuse (S) et lesdites une ou plusieurs valeurs d'une ou de plusieurs caractéristiques optiques dudit filtre optique (OF) comprennent des valeurs quantifiant la transmission de lumière au travers dudit filtre optique (OF).

3. Dispositif selon l'une quelconque des revendications 1 et 2, dans lequel lesdits un ou plusieurs processeurs (12) sont programmés pour déterminer un spectre dudit faisceau lumineux transmis à l'œil (E) du sujet au travers dudit filtre optique (OF) pour la détermination desdites une ou plusieurs valeurs d'une ou de plusieurs caractéristiques spectrales dudit faisceau lumineux transmis à l'œil du sujet au travers dudit filtre optique.

4. Dispositif selon l'une quelconque des revendications 1 à 3, dans lequel lesdites une ou plusieurs mémoires (11) ont en outre en mémoire des données relatives à une ou plusieurs caractéristiques optiques de l'œil (E) du sujet et lesdits un ou plusieurs processeurs sont programmés pour tenir compte de ces données pour la détermination d'une ou de plusieurs valeurs d'une ou de plusieurs caractéristiques spectrales dudit faisceau lumineux transmis à l'œil (E) du sujet au travers dudit filtre optique (OF).

5. Dispositif selon la revendication 4, dans lequel lesdites une ou plusieurs mémoires (11) ont en mémoire des facteurs de pondération (Wf) représentant une sensibilité à la lumière de la rétine de l'œil (E) du sujet à une ou plusieurs longueurs d'onde et dans lequel lesdits un ou plusieurs processeurs (12) sont programmés pour déterminer lesdites une ou plusieurs valeurs d'une ou de plusieurs caractéristiques spectrales dudit faisceau lumineux transmis à l'œil (E) du sujet au travers dudit filtre optique (OF) compte tenu de ces facteurs de pondération (Wf).

6. Dispositif selon l'une quelconque des revendications 1 à 5, dans lequel lesdites une ou plusieurs caractéristiques spectrales du faisceau lumineux transmis à l'œil (E) du sujet au travers dudit filtre optique (OF) comprennent une valeur statistique de longueur d'onde représentant la distribution des longueurs d'onde d'un spectre dudit faisceau lumineux transmis à l'œil (E) du sujet au travers dudit filtre optique (OF).

7. Dispositif selon la revendication 6, dans lequel ladite valeur statistique de longueur d'onde représentant la distribution des longueurs d'onde dudit spectre comprend un barycentre des longueurs d'onde dudit spectre.

8. Dispositif selon l'une quelconque des revendications 6 et 7, dans lequel lesdites une ou plusieurs mémoires (11) ont en mémoire des données relatives à une aberration chromatique longitudinale de l'œil en fonction d'une longueur d'onde d'un faisceau lumineux transmis à l'œil (E) et dans lequel ledit modèle de décalage de réfraction comprend une relation entre le décalage de réfraction de l'œil du sujet et l'aberration chromatique longitudinale de l'œil du sujet à ladite valeur statistique de longueur d'onde.

9. Dispositif selon la revendication 8, dans lequel :

   - lesdites une ou plusieurs valeurs d'une ou de plusieurs caractéristiques spectrales initiales de ladite source lumineuse comprennent un spectre initial de ladite lumière émise par ladite source lumineuse (S) ;
   - lesdits un ou plusieurs processeurs (12) sont programmés pour déterminer une valeur statistique de longueur d'onde de référence représentant la distribution des longueurs d'onde d'un spectre de référence d'un faisceau lumineux de référence émis par la source lumineuse (S) et transmis à l'œil du sujet en l'absence du filtre optique (OF) sur la base dudit spectre initial de ladite lumière émise par ladite source lumineuse (S) ; et
   - ledit modèle de décalage de réfraction tient compte de l'aberration chromatique longitudinale de l'œil (E) du sujet à ladite valeur statistique de longueur d'onde de référence.

10. Dispositif selon la revendication 9, dans lequel ledit modèle de décalage de réfraction fournit une valeur de décalage égale à la différence entre l'aberration chromatique longitudinale de l'œil à la valeur statistique de longueur d'onde et l'aberration chromatique longitudinale de l'œil à ladite valeur statistique de longueur d'onde de référence.

11. Dispositif selon l'une quelconque des revendications 1 à 5, dans lequel lesdites caractéristiques spectrales du faisceau lumineux transmis à l'œil (E) du sujet au travers du filtre optique (OF) comprennent un spectre dudit faisceau lumineux transmis à l'œil (E) au travers dudit filtre optique (OF) sur une plage globale prédéterminée de longueurs d'onde.

12. Dispositif selon la revendication 11, dans lequel lesdites une ou plusieurs mémoires (11) ont en mémoire des valeurs de décalage élémentaire ($S_i$) associées à une plage élémentaire prédéterminée de longueurs d'onde ($B_i$), et ledit modèle de décalage de réfraction comprend une relation entre le décalage de réfraction et une somme, pour l'ensemble de ladite plage élémentaire de longueurs d'onde ($B_i$) comprise dans ladite plage globale prédéterminée de longueurs d'onde, desdites valeurs de décalage élémentaire ($S_i$) pondérées par une intégrale du spectre de ladite lumière transmise à l'œil au travers dudit filtre au sein de la plage élémentaire de longueurs d'onde ($B_i$) correspondante.

13. Dispositif selon la revendication 12, dans lequel ledit modèle de décalage de réfraction fournit une valeur de décalage égale à un rapport entre ladite somme et une intégrale dudit spectre de ladite lumière transmise à l'œil (E) au travers dudit filtre optique (OF) au sein de la plage globale prédéterminée de longueurs d'onde.

14. Dispositif selon l'une quelconque des revendications 12 et 13, dans lequel lesdites valeurs de décalage élémentaire ($S_i$) sont déterminées compte tenu d'une base de données comprenant une pluralité de spectres de lumière de contrôle transmis à un œil et les valeurs mesurées de décalage de réfraction correspondantes dans ledit œil, en minimisant la différence entre chaque valeur mesurée de décalage de réfraction et une valeur calculée de décalage de réfraction, ladite valeur calculée du décalage de réfraction étant déterminée à l'aide de ladite relation et du spectre de lumière de contrôle transmis à l'œil auquel correspond ladite valeur mesurée de décalage de réfraction.

**15.** Article de lunetterie adaptatif pour un sujet, comprenant :

- des verres ophtalmiques à puissance variable et/ou filtre variable dont les variations sont commandées par un contrôleur,
- un dispositif selon l'une quelconque des revendications 1 à 14,

dans lequel ledit dispositif est en communication avec ledit contrôleur et ledit contrôleur est programmé pour déterminer une variation de puissance et/ou une variation de teinte desdits verres ophtalmiques compte tenu du décalage de la réfraction de l'œil (E) du sujet déterminé par ledit dispositif.

**16.** Procédé de détermination d'un décalage d'une valeur de réfraction d'un œil (E) d'un sujet éclairé par un faisceau lumineux émis par une source lumineuse (S), ledit décalage étant induit par un filtre optique (OF) au travers duquel ledit faisceau lumineux est transmis à l'œil (E) du sujet, ledit dispositif comprenant une ou plusieurs mémoires (11) et un ou plusieurs processeurs (12), comprenant les étapes suivantes :

- détermination d'une ou de plusieurs valeurs (100) d'une ou de plusieurs caractéristiques spectrales initiales de ladite source lumineuse,
- détermination d'une ou de plusieurs valeurs (200) d'une ou de plusieurs caractéristiques optiques dudit filtre optique, et
- fourniture d'un modèle de décalage de réfraction (300) liant une grandeur associée audit décalage et une ou plusieurs caractéristiques spectrales du faisceau lumineux transmis à l'œil du sujet ;
- détermination (400) d'une ou de plusieurs valeurs d'une ou de plusieurs caractéristiques spectrales dudit faisceau lumineux transmis à l'œil (E) du sujet au travers dudit filtre optique (OF) sur la base desdites une ou plusieurs valeurs d'une ou de plusieurs caractéristiques spectrales initiales de ladite source lumineuse (S) et desdites une ou plusieurs valeurs d'une ou de plusieurs caractéristiques optiques dudit filtre optique (OF) ; et
- détermination (500) dudit décalage de la réfraction de l'œil (E) du sujet sur la base dudit modèle de décalage de réfraction et desdites valeurs des caractéristiques spectrales dudit faisceau lumineux transmis à l'œil (E) du sujet au travers du filtre optique (OF).

# Fig.1

# Fig.2

# Fig.3

# Fig.4

# Fig.5

# Fig.6

# Fig.7

Weighting Factor

Wf(λ)

Wavelength (nm)

# Fig.8

Transmission (%)

Wavelength (nm)

——— Sunlens A ——— Sunlens B ········ Sunlens C - - - - Sunlens D ——–— Sunlens E

——·–· Sunlens F ······· Sunlens G ■—■■ Sunlens H ■—■·■· Sunlens I

# Fig.9

Refraction shift (D) · Measured · Predicted

# Fig.10

Refraction shift (D) · CS · ETDRS · 3000K · 5500K

# Fig.11

# Fig.12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020315448 A